# EUROPEAN PATENT SPECIFICATION

(11) **EP 2 536 676 B1**
(45) Date of publication and mention of the grant of the patent: **04.04.2018**
(21) Application number: 11708112.5
(22) Date of filing: 17.02.2011
(51) Int. Cl.: C07C 17/25, C07C 21/18

(54) **PROCESS FOR PRODUCING 2-CHLORO-3,3,3-TRIFLUOROPROPENE**
VERFAHREN ZUR HERSTELLUNG VON 2-CHLOR-3,3,3-TRIFLUORPROPEN
PROCÉDÉ DE PRÉPARATION DE 2-CHLORO-3,3,3-TRIFLUOROPROPÈNE

(30) Priority: 19.02.2010 US 282494 P
(43) Date of publication of application: 26.12.2012
(73) Proprietor: Daikin Industries, Ltd., Osaka 530-8323 (JP)
(72) Inventor: YAMASHITA, Tsuneo, Settsu-shi Osaka 566-0044 (JP); NOSE, Masatoshi, Settsu-shi Osaka 566-0044 (JP); KATSUKAWA, Kenichi, Settsu-shi Osaka 566-0044 (JP)
(74) Representative: Hoffmann Eitle
(86) International application number: PCT/JP2011/054055
(87) International publication number: WO 2011/102538

(56) References cited:
- WO-A1-03/027051
- WO-A1-2008/054781
- WO-A1-2009/015317
- WO-A2-2007/079431
- WO-A2-2008/030440
- HASZELDINE R N: "Reactions of fluorocarbon radicals. V. Alternative syntheses for (trifluoromethyl)acetylene (3,3,3-trifluoropropyne) and the influence of polyfluoro groups on adjacent hydrogen and halogen atoms", JOURNAL OF THE AMERICAN CHEMICAL SOCIETY, AMERICAN CHEMICAL SOCIETY, WASHINGTON, DC; US, 1 January 1951 (1951-01-01), pages 2495-2504, XP002503499, ISSN: 0002-7863 cited in the application
- M MAKOSZA: "Reaction of polyfluoroalkenes with diphenylacetonitrile carbanion in a two-phase system", JOURNAL OF FLUORINE CHEMISTRY, vol. 24, no. 4, 1 April 1984 (1984-04-01), pages 387-398, XP55009243, ISSN: 0022-1139, DOI: 10.1016/S0022-1139(00)83158-9
- HASZELDINE: "676. Fluoro-olefins. Part II. Synthesis and reactions of some 3 : 3 : 3-trihalogenopropenes", JOURNAL OF THE CHEMICAL SOCIETY, CHEMICAL SOCIETY, LETCHWORTH; GB, 1 January 1953 (1953-01-01), pages 3371-3378, XP009096268, ISSN: 0368-1769, DOI: 10.1039/JR9530003371 cited in the application

## Description

### Technical Field

The present invention relates to a process for producing 2-chloro-3,3,3-trifluoropropene.

### Background Art

2-Chloro-3,3,3-trifluoropropene (HCFO-1233xf) of the formula CF₃CCl=CH₂ is a useful compound as an intermediate for producing various fluorocarbons, and also as a monomer component of various kinds of polymers. The possibility of using 2-chloro-3,3,3-trifluoropropene as a blowing agent or a propellant has also been suggested.

A known process for preparing HCFO-1233xf comprises reacting anhydrous hydrogen fluoride (HF) in a gas phase in the presence of a catalyst. For example, WO 2007/079431 discloses a process comprising fluorination of 1,1,2,3-tetrachloropropene (HCO-1230xa, CCk₂=CClCH₂Cl) in a gas phase in the presence of a chromium-based catalyst. WO 2008/054781 also reports a process comprising fluorination of 1,1,2,3-tetrachloropropene in a gas phase in the presence of a chromium-based catalyst. Further, WO 2009/015317 teaches that 1,1,2,3-tetrachloropropene (HCO-1230xa), 1,1,1,2,3-pentachloropropane (HCC-240db), 2,3,3,3-tetrachloropropene (HCC-1230xf), etc. can be fluorinated using a stabilizer for minimizing catalyst deterioration.

However, the processes disclosed in the above literatures suffer from various disadvantages. For example, further improvement in the yield of HCFO-1233xf is required, the use of a catalyst is costly, and many products are produced by the reaction in addition to the target product, i.e., HCFO-1233xf, resulting in unsatisfactory selectivity. Further, since catalytic activity tends to decrease as a reaction proceeds, many attempts, such as using a stabilizer for the purpose of minimizing catalyst deactivation, have been made.

R.N. Haszeldine, J. Am. Chem. Soc., 2495-2504 (1951) teaches a process for producing HCFO-1233xf, comprising subjecting 1,2-dichloro-3,3,3-trifluoropropane (HCFC-243db) to a dehydrochlorination reaction in an alkaline solution using an airtight container. However, this process is problematic because it takes a long time for the reaction to be completed, the yield is as low as about 50%, and the production efficiency is unsatisfactory.

Further, a method comprising subjecting 1,1,1,2,3-pentachloropropane (HCC-240db) as a starting material to dehydrochlorination in a solution containing an alcohol and an aqueous alkali metal hydroxide solution to produce 1,1,1,2-tetrachloropropene, and subjecting the produced 1,1,1,2-tetrachloropropene to fluorination using SbF₃, has also been reported (see R.N. Haszeldine, J. Chem. Soc., 3371-3378 (1953)). However, in this process, the use of highly corrosive SbF₃ as a fluorinating agent requires a specific reactor; in addition, because SbF₃ is used in one equivalent per equivalent of HCFO-1233xf, a large amount of SbCl₃ is generated as a by-product. Therefore, in order to recycle SbCl₃ as SbF₃, a fluorination treatment with hydrogen fluoride is required. Because of this, the operation becomes complicated, and thus is not appropriate for industrial production.

As described above, an economically suitable process for readily producing HCFO-1233xf at a high yield has not yet been accomplished at present.

### Summary of Invention

### Technical Problem

The present invention has been accomplished in view of the foregoing problems found in the prior art. A main object of the present invention is to provide a novel process that can produce HCFO-1233xf at a high yield under industrially advantageous conditions.

### Solution to Problem

The present inventors conducted extensive research to achieve the above object. As a result, they found that when a fluorine-containing alkane represented by a specific formula is used as a starting material and mixed with an aqueous solution containing an alkali metal hydroxide or an alkali earth metal hydroxide in a liquid phase in the presence of a catalyst to perform a dehydrohalogenation reaction, the reaction is allowed to proceed at a relatively low temperature, and the target HCFO-1233xf can be obtained at a very high yield. The present inventors found that the above-described process is greatly advantageous for industrial purposes. Thereby, the present invention was accomplished.

Specifically, the present invention provides a process for producing 2-chloro-3,3,3-trifluoropropene (HCFO-1233xf; CF₃-CCl=CH₂) comprising mixing in a liquid state a compound of the formula CF₃-CHCl-CH₂X, wherein X is halogen, with an aqueous solution containing at least one metal hydroxide selected from alkali metal hydroxides and alkali earth metal hydroxides in the presence of at least one catalyst selected from quaternary ammonium salts and aprotic solvents to dehydrohalogenate the CF₃-CHCl-CH₂X, wherein the dehydrohalogenation is continuously performed while the produced CF₃-CCl=CH₂ is collected by distillation.

Preferred embodiments of the invention are as defined in the appended dependent claims and/or in the following detailed description.

Hereinafter, the production process of the present invention is described in detail.

### Starting Compound

According to the present invention, a fluorine-containing alkane of the formula CF₃CHClCH₂X, wherein X is halogen, is used as a starting material. This fluorine-containing alkane is a known compound that can be easily obtained. In the above-mentioned formula, F, Cl, Br and I can be exemplified as the halogen.

### Reaction Process

In the production process of the present invention, a fluorine-containing alkane of the above formula is used in a liquid state as a starting compound. The process comprises mixing this fluorine-containing alkane with an aqueous solution containing at least one metal hydroxide selected from alkali metal hydroxides and alkali earth metal hydroxides to perform a dehydrohalogenation reaction of the starting compound by a liquid phase reaction in a two-phase reaction system.

In the present process, for example, potassium hydroxide, sodium hydroxide, or cesium hydroxide can be used as the alkali metal hydroxide. As examples of the alkali earth metal hydroxide, calcium hydroxide, magnesium hydroxide, barium hydroxide or strontium hydroxide can be used. The alkali metal hydroxides and alkali earth metal hydroxides mentioned above may be used alone, or in a combination of two or more.

There is no particular limitation to the concentration of metal hydroxide in the aqueous solution containing at least one metal hydroxide selected from alkali metal hydroxides and alkali earth metal hydroxides. Generally, aqueous solutions having a concentration of 5 wt.-% to saturation can be used. In particular, when an aqueous solution having a concentration of t 20-50 wt.-% is used, the specific gravity of the aqueous solution will be close to that of a fluorine-containing alkane, which is used as a starting material. This achieves excellent dispersibility therebetween, allowing the reaction to efficiently proceed, and enabling a reduction in the amount of the below-mentioned catalyst used.

The amount of the aqueous solution containing a metal hydroxide may be adjusted so that the amount of the metal component contained therein is 1-1.5 equivalents, per equivalent of a fluorine-containing alkane of the formula CF₃CHClCH₂X, used as a starting material. Specifically, when a hydroxide of an alkali metal, which is a monovalent metal, is used as the metal hydroxide, an aqueous solution may contain the alkali metal hydroxide in an amount of 1-1.5 mol, per 1 mol of the fluorine-containing alkane of the formula CF₃CHClCH₂X. When a hydroxide of an alkaline earth metal, which is a divalent metal, is used as the metal hydroxide, an aqueous solution may contain the alkali earth metal hydroxide in an amount of 0.5-0.75 mol, per 1 mol of the fluorine-containing alkane of the formula CF₃CHClCH₂X.

In the present process, the fluorine-containing alkane of the formula CF₃CHClCH₂X is mixed with an aqueous solution containing at least one metal hydroxide selected from alkali metal hydroxides and alkali earth metal hydroxides in the presence of a catalyst. This allows a dehydrohalogenation reaction of the fluorine-containing alkane to proceed at the interface between the fluorine-containing alkane phase and a water phase containing the metal hydroxide, thereby producing the target 2-chloro-3,3,3-trifluoropropene (HCFO-1233xf).

The catalyst is at least one catalyst selected from phase-transfer catalysts selected from quaternary ammonium salts, and aprotic polar solvents. By performing the aforementioned dehydrohalogenation reaction in the presence of such a catalyst, the target 2-chloro-3,3,3-trifluoropropene (HCFO-1233xf) can be produced as a result of the liquid phase reaction, at a high yield in a shorter period of time and at a relatively low reaction temperature.

Examples of quaternary ammonium salts as phase-transfer catalysts include, but are not particularly limited to, tetrabutylammonium bromide (TBAB), trimethylbenzylammonium bromide, triethylbenzylammonium bromide and trioctylmethylammonium chloride (TOMAC).

There is no limitation to the aprotic polar solvents, as long as they have a polarity and no active protons. Examples thereof include tetrahydrofuran, 1,2-dimethoxyethane, propylene carbonate, acetonitrile, dimethylformamide, dimethyl sulfoxide, bis(2-methoxyethyl)ether, 1,4-dioxane, diethyl ether and diisopropyl ether.

These catalysts may be used alone, or in a combination of two or more. Of these, trioctylmethylammonium chloride (TOMAC), which is a phase-transfer catalyst, is particularly preferable.

The amount of the catalyst is not particularly limited. It is preferable that a phase-transfer catalyst is used in an amount of 0.3-5 parts by weight (pbw), and that an aprotic polar solvent is used in an amount of 10-50 pbw, based on 100 pbw of the fluorine-containing alkane of the formula CF₃CHClCH₂X.

According to the present process, by mixing an aqueous solution containing at least one metal hydroxide selected from alkali metal hydroxides and alkali earth metal hydroxides, a fluorine-containing alkane of the formula CF₃CHClCH₂X, and a catalyst, a dehydrohalogenation reaction of the fluorine-containing alkane is allowed to proceed. These components are added in an arbitrary order. Further, there is no limitation to the stirring method; a method by which each component can be homogeneously mixed may be appropriately employed. The reaction can proceed by, for example, causing a sufficient mechanical stirring of an aqueous solution containing a metal hydroxide and a catalyst, and adding the fluorine-containing alkane of the formula CF₃CHClCH₂X dropwise to the resulting aqueous solution.

The reaction temperature may be adjusted to a temperature range in which both the aqueous metal hydroxide solution and the fluorine-containing alkane can exist as a liquid, generally 0-30°C. According to the present invention, the target HCFO-1233xf can be obtained at a high yield at the aforementioned relatively low reaction temperature.

In the present process, a reaction apparatus in which a distillation column is attached to a reactor for mixing an aqueous solution containing a metal hydroxide, a fluorine-containing alkane, and a catalyst is used. With this reaction apparatus, HCFO-1233xf is continuously produced by using the starting materials in liquid state, and by performing a dehydrohalogenation reaction at a temperature exceeding about 14°C, which is a boiling point of target HCFO-1233xf, for example, at 14-30°C, and continuously isolating and collecting the produced HCFO-1233xf by distillation. In particular, it is preferable that this reaction be performed at a temperature of 14-20°C. An excessively high reaction temperature is not preferable because the starting materials are easily incorporated into the reaction product collected by distillation. In contrast, an excessively low reaction temperature is also not preferable because the reaction product is dissolved in the reaction solution, which allows the reaction to further proceed, producing a by-product, i.e., a propyne compound, which is easily incorporated into the reaction product collected by distillation.

In the above-mentioned method, HCFO-1233xf can be continuously produced by appropriately adding to the reactor the fluorine-containing alkane and the metal oxide consumed during the reaction.

### Recycling of Reaction Solution

In the production process of the present invention, a metal halide is produced as a by-product, other than the target HCFO-1233xf. Generally, a metal halide is low in solubility compared with the metal hydroxide used as a starting material, and will thus be precipitated in a reaction solution. For example, the solubility of KOH (20°C) is 110 g/100 cc, whereas the solubility of KCl is 34 g/100 cc.

In the production process of the present invention, the precipitate of a metal halide, such as the produced KCl, or the like, is separated by filtration from the reaction solution. Thereafter, a fluorine-containing alkane and a metal hydroxide, which are used as starting materials, are added to this reaction solution to readjust the concentrations of these components. Thereby, HCFO-1233xf can be continuously produced. In this way, the catalyst contained in the reaction solution can be effectively used, and the amount of waste fluid can be greatly reduced. Further, the metal halide collected by filtration can also be better utilized.

### Advantageous Effects of Invention

The production process of the present invention can be performed in a liquid phase at a relatively low temperature without using a catalyst that is difficult to handle, and can produce the target HCFO-1233xf at a high yield.

For this reason, the method of the present invention is industrially advantageous as a production process of HCFO-1233xf.

### Description of Embodiments

Hereinafter, the present invention is described in more detail with reference to Examples.

### Example 1

A 50 wt% aqueous potassium hydroxide solution (1,000 g) and Aliquat 336 (tradename, produced by Aldrich) (trioctylmethylammonium chloride (TOMAC)) (3.0 g), which is a phase-transfer catalyst, were added to a 1-liter three-necked flask equipped with a thermometer for measuring the reactor temperature, a thermometer for measuring the temperature at the top of rectification column, a dropping funnel, an Oldershaw-type rectification column (five plates), a rectification head, a cold finger trap using dry ice/acetone, and a receiver. The resulting mixture was cooled in a water bath to 10°C. While stirring the cooled mixture with a magnetic stirrer, 1,2-dichloro-3,3,3-trifluoropropane (HCFC-243db) (700 g, 4.2 mol) was added dropwise from a dropping funnel so that the temperature inside the reactor maintained 20°C or lower. When the temperature inside the reactor reached 14°C or higher, gas was generated. When the temperature at the top of the rectification column reached 13°C, the reflux ratio (return: distillate) was changed to 2:1 from the total reflux. The distillate was collected until the temperature at the top of the rectification column reached 15°C. Thereby, 530 g of the fraction containing the target 2-chloro-3,3,3-trifluoropropene (HCFO-1233xf) was obtained. The purity as measured by a gas chromatographic analysis was 99.2%; thus, the yield at this time was 97%.

### Example 2

The same reaction was performed in the same manner as in Example 1, except that 220 g (1.32 mol) of HCFC-243db, 95 g of 60 wt% aqueous sodium hydroxide solution, and 1.0 g of Aliquat 336 (produced by Aldrich) were used. Thereby, 174 g of a fraction containing the target HCFO-1233xf was obtained. The purity as measured by a gas chromatographic analysis was 99.1%; thus, the yield at this time was 96%.

### Example 3

The same reaction was performed in the same manner as in Example 1, except that 180 g (1.08 mol) of HCFC-243db, 280 g of 50 wt% aqueous potassium hydroxide solution, and, in place of the phase-transfer catalyst, 30.0 g of dimethylacetamide, which is an aprotic polar solvent, were used. Thereby, 108 g of a fraction containing HCFO-1233xf was obtained. The purity as measured by a gas chromatographic analysis was 99.0%; thus, the yield at this time was 89%.

### Example 4

The residue in the three-necked flask, obtained after the reaction of Example 1 was concentrated, and the precipitated KCl was separated by filtration under reduced pressure. The amount of the filtrate at this time was 250 g, and Aliquat 336, which is a phase-transfer catalyst, remained in the filtrate. A 60 wt% aqueous KOH solution (750 g) was added to the resulting filtrate, and the resulting mixture was cooled in a water bath to 10°C, followed by a reaction as in Example 1 using HCFC-243db (700 g, 4.2 mol). Thereby, 525 g of a fraction containing the target HCFO-1233xf was obtained. The purity as measured by a gas chromatographic analysis was 99.4%; thus, the yield at this time was 95.2%.

## Claims

1. A process for producing 2-chloro-3,3,3-trifluoropropene (CF₃-CCl=CH₂) comprising mixing in a liquid state a compound of the formula CF₃-CHCl-CH₂X, wherein X is halogen, with an aqueous solution containing at least one metal hydroxide selected from alkali metal hydroxides and alkali earth metal hydroxides in the presence of at least one catalyst selected from quaternary ammonium salts and aprotic solvents to dehydrohalogenate the CF₃-CHCl-CH₂X, wherein the dehydrohalogenation is continuously performed while the produced CF₃-CCl=CH₂ is collected by distillation.

2. The process of Claim 1, wherein the reaction is performed at a temperature of 0-30°C.

3. The process of claim 1 or 2, which further comprises
(i) removing precipitates in a reaction solution obtained in the process of any of claims 1-2,
(ii) adding a compound of the formula CF₃-CHCl-CH₂X, wherein X is halogen, and at least one metal hydroxide selected from alkali metal hydroxides and alkali earth metal hydroxides to the reaction solution, and
(iii) dehydrohalogenating the CF₃-CHCl-CH₂X by the process of any of Claims 1-2.

## Patentansprüche

1. Verfahren zur Herstellung von 2-Chlor-3,3,3-trifluorpropen (CF₃-CCl=CH₂), umfassend das Mischen einer Verbindung der Formel CF₃-CHCl-CH₂X, worin X ein Halogen ist, in flüssigem Zustand mit einer wässrigen Lösung, die mindestens ein Metallhydroxid enthält, ausgewählt aus Alkalimetallhydroxiden und Erdalkalimetallhydroxiden, in Gegenwart mindestens eines Katalysators, ausgewählt aus quartären Ammoniumsalzen, und eines aprotischen Lösungsmittels, so dass CF₃-CHCl-CH₂X dehydrohalogeniert wird, worin die Dehydrohalogenierung kontinuierlich durchgeführt wird, während das hergestellte CF₃-CCl=CH₂ mittels Destillation gesammelt wird.

2. Verfahren gemäß Anspruch 1, worin die Reaktion bei einer Temperatur von 0-30°C durchgeführt wird.

3. Verfahren gemäß Anspruch 1 oder 2, das ferner umfasst
(i) Entfernen von Niederschlägen in einer Reaktionslösung, die im Verfahren gemäß Anspruch 1 oder 2 erhalten wurde,
(ii) Zugeben einer Verbindung der Formel CF₃-CHCl-CH₂X, worin X Halogen ist, und mindestens eines Metallhydroxids, ausgewählt aus Alkalimetallhydroxiden und Erdalkalimetallhydroxiden, zur Reaktionslösung und
(iii) Dehydrohalogenieren des CF₃-CHCl-CH₂X mittels des Verfahrens gemäß Anspruch 1 oder 2.

## Revendications

1. Procédé de production de 2-chloro-3,3,3-trifluoropropène (CF₃-CCl=CH₂) comprenant le mélange dans un état liquide d'un composé de formule CF₃-CHCl-CH₂X, dans laquelle X est un halogène, avec une solution aqueuse contenant au moins un hydroxyde métallique sélectionné parmi les hydroxydes de métaux alcalins et les hydroxydes de métaux alcalino-terreux en présence d'au moins un catalyseur sélectionné parmi les sels d'ammonium quaternaire et les solvants aprotiques pour déshydrohalogéner le CF₃-CHCl-CH₂X, dans lequel la déshydrohalogénation est réalisée en continu tandis que le CF₃-CCl=CH₂ produit est collecté par distillation.

2. Procédé selon la revendication 1, dans lequel la réaction est réalisée à une température de 0 à 30 °C.

3. Procédé selon la revendication 1 ou 2, qui comprend en outre
(i) l'élimination des précipités dans une solution réactionnelle obtenue dans le procédé selon l'une quelconque des revendications 1 et 2,
(ii) l'ajout d'un composé de formule CF₃-CHCl-CH₂X, dans laquelle X est un halogène, et d'au moins un hydroxyde métallique sélectionné parmi les hydroxydes de métaux alcalins et les hydroxydes de métaux alcalino-terreux à la solution réactionnelle, et
(iii) la déshydrohalogénation du CF₃-CHCl-CH₂X par le procédé selon l'une quelconque des revendications 1 et 2.
